# EUROPEAN PATENT APPLICATION

(11) **EP 4 599 811 A1**
(43) Date of publication of application: **13.08.2025**
(21) Application number: 24218249.1
(22) Date of filing: 09.12.2024
(51) Int. Cl.: A61F 13/62

(54) **A CLOSING ELEMEN AND METHOD FOR PRODUCING ABSORBENT SANITARY ARTICLES AND RELATED ABSORBENT SANITARY ARTICLE**

(30) Priority: 08.02.2024 IT 202300026724
(71) Applicant: Fameccanica.Data S.p.A., 66020 San Giovanni Teatino (CH) (IT)
(72) Inventor: SABLONE, Gabriele, 66020 San Giovanni Teatino (IT)
(74) Representative: Buzzi, Notaro & Antonielli d'Oulx S.p.A.

(57) **Abstract**

A closing element for producing reclosable absorbent sanitary articles, comprising a non-woven web (12) folded according to a general omega-shaped configuration and including a base (14), two intermediate flaps (18) connected to respective ends of the base (14) by respective first folds (20), two distal flaps (24) connected to respective ends of the respective intermediate flaps (18) by respective second folds (26), wherein the closing element (10) comprises surface fastening elements (30) arranged on respective surfaces (44) of the distal flaps (24) facing respective intermediate flaps (18).

## Description

### Field of the invention

The present invention relates to a closing element intended to be used for producing absorbent sanitary articles.

The invention was developed in particular for producing reclosable absorbent sanitary articles, such as diapers, diaper-pants, absorbent articles for incontinent adults, etc.

The invention may be used in particular in production processes called "Cross Direction".

The invention also relates to an absorbent sanitary article produced using such a closing element.

According to another aspect, the invention concerns a machine for producing absorbent sanitary articles.

### Prior art

An established technique for producing reclosable absorbent sanitary articles involves forming a continuous composite web comprising at least one continuous waistband, parallel to the machine direction, and a plurality of absorbent cores attached to the continuous waistband and extending orthogonally to the machine direction. In certain cases, two continuous waistbands parallel to the machine direction are used, between which the absorbent cores are attached. Sometimes the production process involves the use of a single continuous waistband parallel to the machine direction and to which the ends of absorbent cores extending orthogonally to the machine direction are attached.

This production technique called Cross Direction is different from the production technique called Machine Direction, which involves producing absorbent sanitary articles while they advance with their longitudinal axes parallel to the machine direction.

Examples of methods for producing absorbent sanitary articles according to the Cross Direction technique are described in documents EP-A-1013251, IT1379452, IT1410464 and IT1410465 by the same Applicant.

For producing reclosable absorbent sanitary articles, refastenable closing elements are applied to one of the continuous waistbands spaced apart in the machine direction.

EP1523968-A by the same applicant describes a closing element for producing absorbent sanitary articles wearable in the form of panties, comprising a non-woven web folded according to a general omega-shaped configuration and including a base divided into two sections located on opposite sides of a central plane, two intermediate flaps located on opposite sides of the central plane, connected to respective outer ends of the base sections and permanently fixed to the base, two distal flaps located on opposite sides of the central plane, connected to respective inner ends of the respective intermediate flaps by respective folds, and fixed in a separable manner to respective intermediate flaps. The base of the closing element is fixed to a waistband of a sanitary article. Respective surface fasteners are fixed on surfaces of respective distal flaps opposite to the base.

One of the problems with this solution is that the surface fastening elements (e.g. Velcro^{©} type micro-hook elements) are exposed on one surface of the waistband during the manufacturing process.

This solution is suitable when absorbent sanitary articles are closed like panties before packaging. In this case, during the production process, the absorbent sanitary articles are folded in such a way that the surface fastening elements of the closing elements come into contact with corresponding closing elements to close the absorbent sanitary articles in the manner of panties.

However, when absorbent sanitary articles are intended to be packaged in an open configuration and to be closed when the article is worn, exposed surface fasteners on the waistbands tend to stick to any fabric or similar surfaces both during the manufacturing and packaging process and when the article is worn.

US2003078558A1 describes an absorbent sanitary article having a rear waistband provided with two closing elements each of which comprises a non-woven fabric backing having a distal portion provided with micro-hook fasteners and a proximal portion that is permanently attached to the outer surface of the rear waistband. Each of the closing elements may be folded n number of times, and the folds are held in place by glue or thermal or ultrasonic welding. To avoid contact between the micro-hook material and the wearer's clothing, the micro-hook fixing portion may be folded inward, so that the micro-hook surface adheres to the surface of the non-woven fabric backing.

The main drawback of the solution according to US2003078558A1 is that in the production process, two separate closing elements must be applied to each absorbent sanitary article, which complicates the apparatus and the production process.

### Object and summary of the invention

The object of the present invention is to provide a closing element that overcomes the problems of the prior art.

According to the present invention, this object is achieved by a closing element having the characteristics forming the subject of claim 1.

According to another aspect, the invention relates to a method for producing absorbent sanitary articles having the characteristics forming the subject of claim 7 and a machine for producing absorbent sanitary articles according to claim 10.

Preferred embodiments form the subject of the dependent claims.

The claims form an integral part of the disclosure provided here in relation to the invention.

### Brief description of the drawings

The present invention will now be described in detail with reference to the attached drawings, given purely by way of non-limiting example, in which:
- Figure 1 is a perspective view of a closing element according to the present invention,
- Figure 2 is a schematic front view of the closing element of Figure 1, and
- Figure 3 is a schematic plan view illustrating a step of a method for producing absorbent sanitary articles,
- Figures 4 and 5 are schematic front views of the part indicated by the arrow IV in Figure 3 in two successive steps of the method for producing absorbent sanitary articles,
- Figures 6 and 7 are schematic plan views of an absorbent sanitary article, with side closures in the closed and open positions, respectively,
- Figure 8 is a schematic cross-section along line VIII-VIII of Figure 7, and
- Figures 9-13 are schematic plan views illustrating different embodiments of side closures indicated by arrow IX in Figure 7.

### Detailed description

With reference to the Figures, numeral 10 indicates a closing element for producing reclosable absorbent sanitary articles.

The closing elements 10 are intended to be fixed at spaced apart positions on a continuous elastic band in a process for producing absorbent sanitary articles according to the technique called Cross Direction, as described in EP-A-1523968 by the same applicant.

The closing element 10 comprises a web of non-woven fabric 12 folded according to a general omega-shaped configuration so as to form a base 14, two intermediate flaps 18 and two distal flaps 24.

The base 14 is generally flat and is divided into two sections 16 located on opposite sides with respect to a central plane X perpendicular to the base 14.

The two intermediate flaps 18 are parallel to the base 14 and are located on opposite sides of the central plane X. The intermediate flaps 18 are connected to respective ends of the base 14 by respective first folds 20. The intermediate flaps 18 have respective surfaces 40 facing the base 14 and permanently fixed to corresponding surfaces 42 of the base 14, for example, by respective glue layers 22 or, alternatively, by thermal or ultrasonic welding.

The two distal flaps 24 are parallel to respective intermediate flaps 18 and are located on opposite sides of the central plane X. The distal flaps 24 are connected to respective ends of the intermediate flaps 18 by respective second folds 26.

The closing element 10 comprises surface fastening elements 30 arranged on surfaces 44 of the distal flaps 24 facing respective intermediate flaps 18.

The surface fastening elements 30 may be micro-hook fastening elements designed to form a Velcro^{®}-type surface connection with corresponding micro-loop fastening elements.

The surface fasteners 30 may be attached to the surfaces 44 of the distal flaps 24 by glue or by thermal or ultrasonic welding.

The surface fasteners 30 may be integrally formed on the surfaces 44 of the distal flaps 24, for example, by an ultrasonic apparatus of the type described in the patent application EP4108220 by the same Applicant.

For each distal flap 24 the surface fastening elements 30 may be continuous along a direction parallel to the central plane X.

Alternatively, for each distal flap 24 the surface fastening elements 30 may be discontinuous along a direction parallel to the central plane X.

The surface fastening elements 30 may releasably engage surfaces 46 of respective intermediate flaps 18 facing away from the base 14. This is particularly useful in the case wherein the surface fasteners 30 are micro-hook fasteners that engage the surface 46 of the intermediate flaps 18 made of non-woven fabric.

Each of the surface fasteners 30 is contained between a respective first fold 20 and a respective second fold 26. Therefore, the surface fastening elements 30 do not protrude laterally beyond the respective first folds 20, and any contact between the surface fastening elements 30 and elements external to the closing element 10 is thus avoided.

The distal flaps 24 may have respective ends 48 that protrude laterally beyond corresponding outer ends 50 of respective surface fasteners 30. This makes it easier to grip the ends 48 with one's fingers to open the closing element 10.

In possible embodiments, the ends 48 of the distal flaps 24 may protrude laterally outwards beyond respective second folds 26 in order to further facilitate gripping of the ends 48 with the fingers.

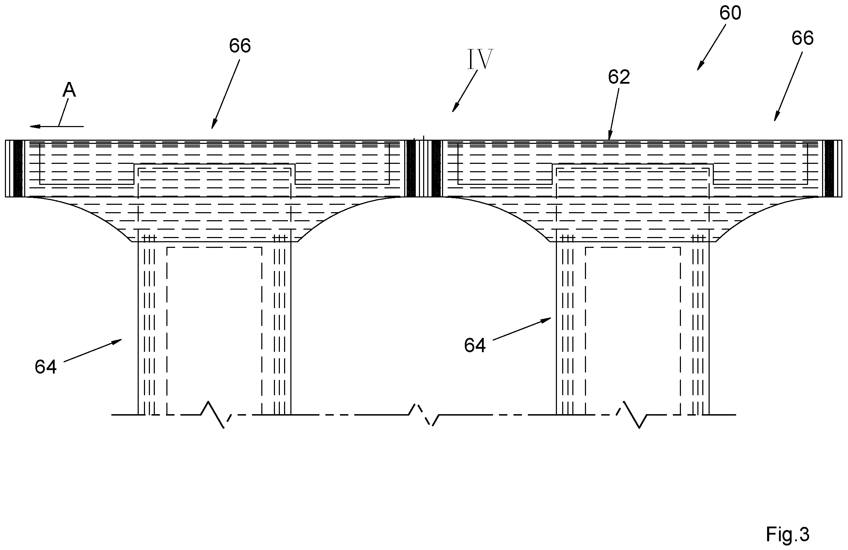

Figure 3 schematically illustrates a step of a method for producing absorbent sanitary articles.

The method involves forming a continuous composite tape 60 advancing in a machine direction A. The continuous composite tape 60 is formed of a continuous array of absorbent sanitary article blanks 66.

The continuous composite tape 60 comprises a continuous waistband 62 parallel to the machine direction A. The continuous waistband 62 is typically elastic and may comprise a plurality of elastic threads attached between two nonwoven webs.

The continuous composite tape 60 comprises a plurality of absorbent cores 64 having respective ends attached to the continuous waistband 62. The absorbent cores 64 extend orthogonally to the machine direction A and are spaced apart from each other in the machine direction A.

The absorbent sanitary article blanks 66 each comprise an absorbent core 64 and a respective continuous waistband section 62.

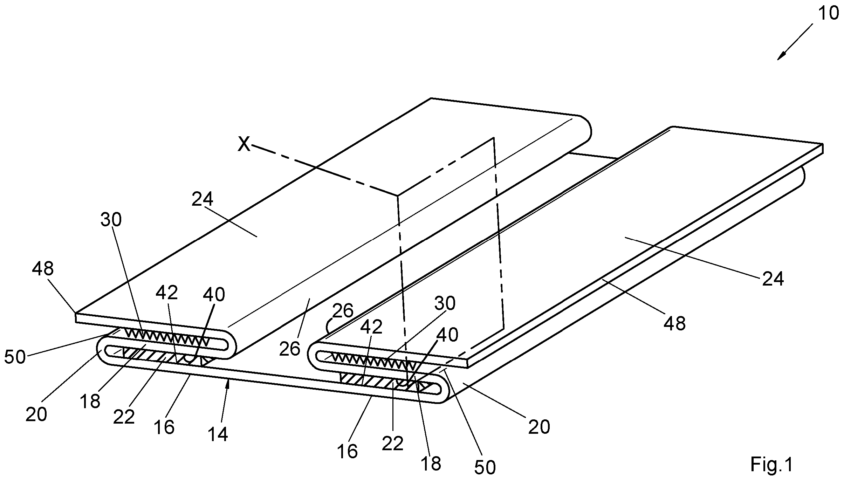

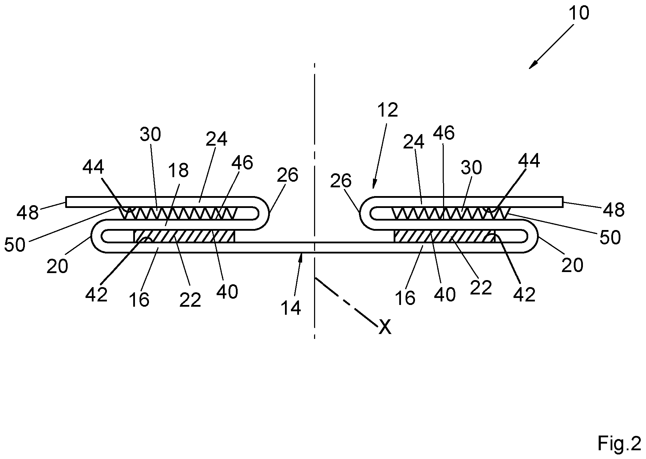

The method involves forming a plurality of fastening elements 10, each having the structure illustrated in Figures 1 and 2. The fastening elements 10 may be produced starting from a continuous non-woven web, on two side regions of which respective continuous or intermittent strips of micro-hook material forming the fastening elements 30 are attached or integrally formed. Glue layers 22 are also applied to the continuous non-woven web. The continuous non-woven web is folded into an omega shape so as to assume the shape illustrated in Figures 1 and 2. Then, the continuous non-woven web is cut in the transverse direction to form fastening elements 10.

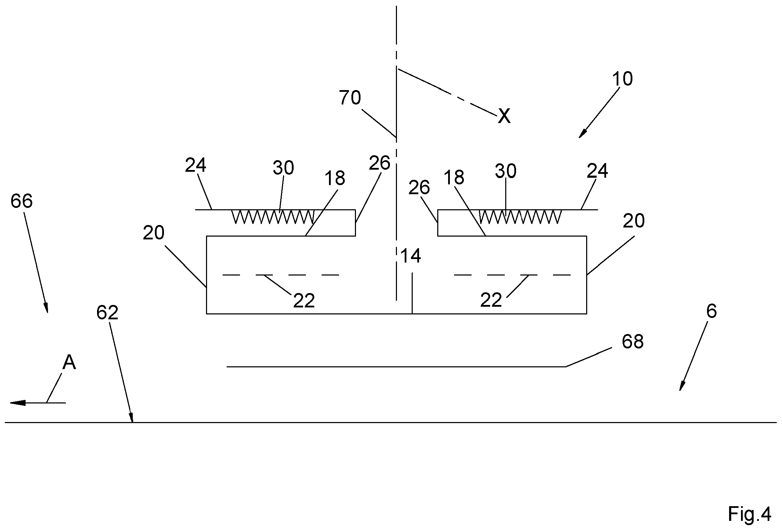

As illustrated in Figures 3 and 4, the bases 14 of the fasteners 10 are attached onto a surface of the continuous waistband 62 at positions spaced apart from each other along the machine direction A. As illustrated schematically in Figure 4, attaching the bases 14 of the fasteners 10 to the continuous waistband 62 may be accomplished, for example, by glue layers 68 applied intermittently on the continuous waistband 62.

The fastening elements 10 are oriented with their respective central planes X perpendicular to the machine direction A. The fastening elements 10 are attached to the continuous waistband 62 so that the central plane X of each closing element 10 is aligned with a separation line between each pair of adjacent absorbent sanitary article blanks 66.

As illustrated in Figure 3, the continuous waistband 62 may have an inner edge 80 with a contoured shape to conform to the wearer's legs. The inner edge 80 may be cut flush with the inner edges of the surface fasteners 30.

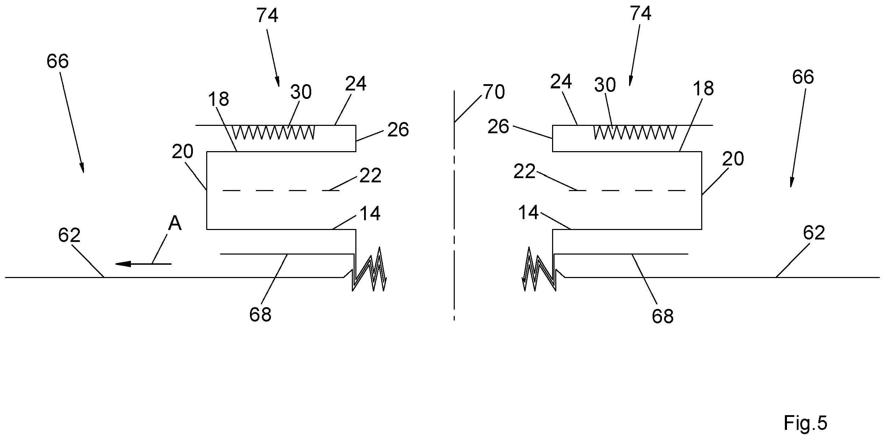

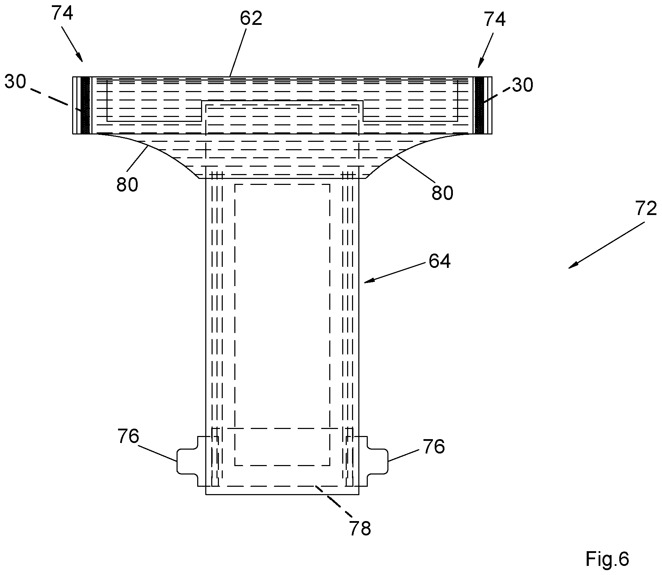

As illustrated in Figures 4 and 5, after attachment of the fasteners 10, the continuous waistband 62 is cut transversely along cutting lines 70 substantially aligned with the central planes X of the fasteners 10. The transverse cut of the continuous waistband 62 along the cutting lines 70 forms absorbent sanitary articles 72 having the shape illustrated schematically in Figure 6. Each cutting line 70 divides a respective closing element 10 into two symmetrical sections, each of which forms a side closure 74 of an absorbent sanitary article 72.

As illustrated in Figure 5, after cutting the continuous waistband 62, the portions of the continuous waistband 62 adjacent to the cutting line 70 elastically retract against their respective side closures 74.

During the formation, fixing and cutting steps of the fasteners 10, the surface fasteners 30 are enclosed between respective distal flaps 24 and intermediate flaps 18 and releasably engage surfaces 46 of respective intermediate flaps 18 facing away from the base 14.

This avoids problems that could arise from the adhesion of the surface fastening elements 30 to materials or components used in the manufacturing or packaging process of the absorbent sanitary articles 72.

At the end of the manufacturing process, absorbent sanitary articles 72 are obtained, each comprising a waistband 62, an absorbent core 64 having one end fixed to the waistband 62, and two side closures 74 attached to opposite ends of the waistband 62, wherein the side closures 74 are formed by respective sections of a closing element 10.

At the end of the production process, the absorbent sanitary articles 72 are folded and wrapped to create product packages.

The absorbent sanitary articles 72 are packaged with the side closures 74 of the absorbent sanitary articles 72 in the closed configuration, in which the surface fastening elements 30 are enclosed between respective distal flaps 24 and intermediate flaps 18.

When in use the surface fasteners 30 are hidden and do not adhere to clothing or other fabric articles or the like when the article is worn.

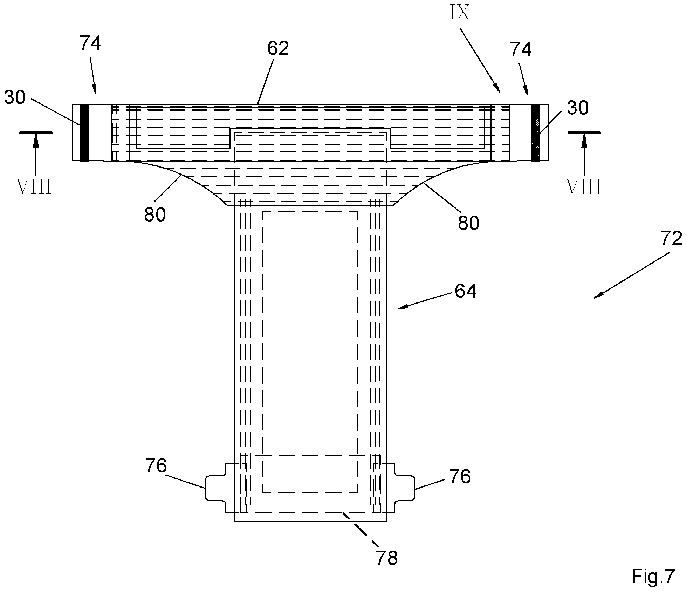

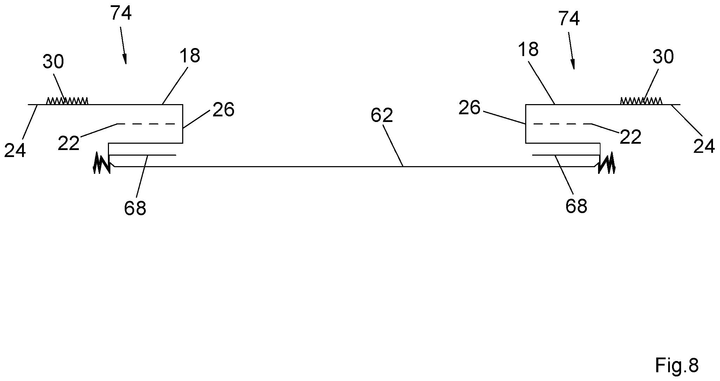

When in use, the side closures 74 are opened as shown in Figures 7 and 8. In this configuration the surface fasteners 30 are coupled with side flaps 76 or a front panel 78 to close the absorbent sanitary article 72 around the waist of the user.

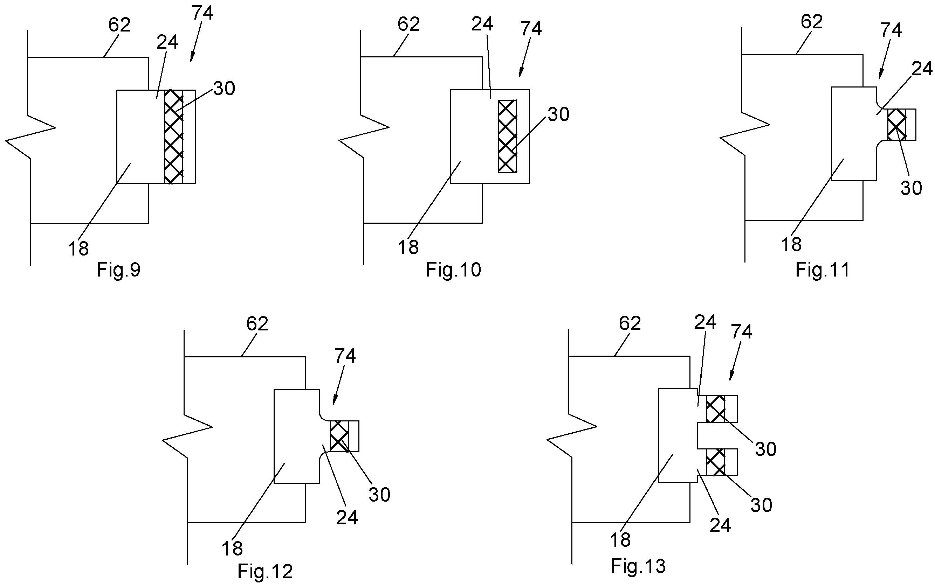

Referring to Figure 9, the surface fastening element 30 may have a width equal to that of the respective distal flap 24.

Alternatively, as illustrated in Figure 10, the surface fastener 30 may have a width smaller than that of the respective distal flap 24 and may be surrounded on four sides by the material of the distal flap 24.

Referring to Figure 11, the distal flap 24 of each side closure 74 may be shaped to have a width smaller than that of the respective intermediate flap 18. The shaping may be asymmetrical as illustrated in Figure 11 (which results in a scrap cut).

With reference to Figures 12 and 13, the distal flap 24 of each side closure 74 may be cut symmetrically so as to form two side closures 74 with complementary shapes, thus avoiding the formation of scraps.

To achieve the embodiments of Figures 11, 12 and 13, it may be advantageous for the base 14 to be formed by two or more webs extending from opposite sides of the central plane X and joined together along a joint line parallel to the central plane X.

Of course, without prejudice to the principle of the invention, the details of construction and the embodiments can be widely varied with respect to those described and illustrated, without thereby departing from the scope of the invention as defined by the claims that follow.

## Claims

1. A closing element for producing reclosable absorbent sanitary articles, comprising a non-woven web (12) folded according to a general omega configuration so as to form:
- a base (14) divided into two sections (16) located on opposite sides with respect to a central plane (X) perpendicular to the base (14),
- two intermediate flaps (18) parallel to the base (14) and located on opposite sides of said central plane (X), wherein the intermediate flaps (18) are connected to respective ends of the base (14) by respective first folds (20) and have respective surfaces permanently fixed to the base (14),
- two distal flaps (24) parallel to the respective intermediate flaps (18) and located on opposite sides of said central plane (X), wherein the distal flaps (24) are connected to respective ends of the respective intermediate flaps (18) by respective second folds (26),
wherein the closing element (10) comprises surface fastening elements (30) arranged on respective surfaces (44) of said distal flaps (24) facing respective intermediate flaps (18).

2. The closing element of claim 1, wherein the surface fastening elements (30) are micro-hook surface fasteners.

3. The closing element of claim 1 or claim 2, wherein the surface fastening elements (30) engage in a releasable manner surfaces (46) of respective intermediate flaps (18) facing away from the base (14).

4. The closing element of any of the preceding claims, wherein each of said surface fastening elements (30) is contained between a respective first fold (20) and a respective second fold (26).

5. The closing element of any of the preceding claims, wherein said distal flaps (24) have respective ends (48) which protrude in a lateral direction beyond corresponding outer ends (50) of respective surface fastening elements (30).

6. The closing element of claim 5, wherein said ends (48) of said distal flaps (24) protrude in a lateral direction outwards beyond respective first folds (20).

7. An absorbent sanitary article comprising a waistband (62), an absorbent core (64) having one end attached to said waistband (62), and two side closures (74) attached to opposite ends of said waistband (62), wherein said side closures (74) are formed by respective sections of a closing element (10) according to any one of claims 1-6.

8. A method for producing absorbent sanitary articles, comprising:
- forming a continuous composite tape (60) advancing in a machine direction (A) formed by a continuous array of absorbent sanitary article blanks (66), wherein the continuous composite tape (60) comprises a continuous waistband (62) parallel to the machine direction (A) and a plurality of absorbent cores (64) having respective ends fixed to the continuous waistband (62),
- forming a plurality of fastening elements (10), according to any one of claims 1-6,
- attaching the bases (14) of the fastening elements (10) onto a surface of the continuous waistband (62) in positions spaced apart from each other along said machine direction (A), and
- cutting transversally said continuous waistband (62) along cutting lines (70) substantially aligned with the central planes (X) of the fastening elements (10).

9. The method according to claim 8, wherein during said steps of forming, fixing and cutting of said fastening elements (10) the surface fastening elements (30) are enclosed between respective distal flaps (24) and intermediate flaps (18).

10. A machine for producing absorbent sanitary articles configured to implement a method according to claim 8 or claim 9.
